# EUROPEAN PATENT SPECIFICATION

(11) **EP 4 291 874 B1**
(45) Date of publication and mention of the grant of the patent: **20.11.2024**
(21) Application number: 22707844.1
(22) Date of filing: 08.02.2022
(51) Int. Cl.: G01N 33/02, G01N 21/84, G01N 21/55, G01N 21/31

(54) **PORTABLE DEVICE FOR ANALYSING VEGETABLE MATRICES ON THE FIELD AND RELATED SYSTEM AND METHOD**
TRAGBARE VORRICHTUNG ZUR ANALYSE PFLANZLICHER MATRIZEN AUF DEM FELD SOWIE ZUGEHÖRIGES SYSTEM UND VERFAHREN
DISPOSITIF PORTABLE D'ANALYSE DE MATRICES VÉGÉTALES SUR LE TERRAIN ET SYSTÈME ET PROCÉDÉ ASSOCIÉS

(30) Priority: 10.02.2021 IT 202100002888
(43) Date of publication of application: 20.12.2023
(73) Proprietor: UNIVERSITA' DEGLI STUDI DI MILANO, 20122 Milano (IT); Officina Delle Soluzioni SNC di Bonino Michelino, Molteni Federico e Torta Fabio Fiscalmente Siglabile "Odiesse SNC", 12062 Cherasco (Cuneo) (IT)
(72) Inventor: GUIDETTI, Riccardo, 20131 MILANO (IT); BEGHI, Roberto, 20161 MILANO (IT); GIOVENZANA, Valentina, 20900 MONZA (MONZA-BRIANZA) (IT); TUGNOLO, Alessio, 21055 Gorla Minore (VARESE) (IT); CASSON, Andrea, 20080 Cisliano (MILANO) (IT); PAMPURI, Alessia, 20054 Segrate (MILANO) (IT); TORTA, Fabio, 12062 Cherasco (CUNEO) (IT); MOLTENI, Federico, 21040 Venegono Inferiore (VARESE) (IT); BONINO, Michelino, 12040 Vezza d'Alba (CUNEO) (IT)
(74) Representative: Penza, Giancarlo
(86) International application number: PCT/IB2022/051110
(87) International publication number: WO 2022/172153

(56) References cited:
- WO-A1-2019/236843
- US-A1- 2010 182 604

## Description

### Technical field

The subject of the present invention is a low-cost portable device for analysing vegetable matrices in the field, a method and a system for analysing vegetable matrices.

Specifically, the sectors in which the invention is applicable concern the agri-food, processing, agricultural, wine, olive oil, logistics, catering and animal feed sectors.

The device of the present invention addresses the needs of monitoring and control of agri-food chains from the field to the processing stages up to the product ready for consumption (for example application directly at the point of sale).

Some non-limiting examples of application of the device according to the present invention comprise vegetable matrices such as fruits, e.g., grapes, blueberry, tomato or vegetables such as salad or olives.

### State of the art

Given the need to have information available in a short time in order to better manage the control stages of the crop productions, a substantial change in the measuring methods has been taking place in recent years.

In this perspective, the opportunity to replace or flank the classical techniques of investigation, based on traditional laboratory analyses, with methods mainly based on physical approaches of rapid execution, limited invasiveness, high environmental sustainability, applicable directly in the field and that would allow the creation of databases of objective information characterizing the sample, which are particularly large and shared through data clouds, is becoming increasingly popular.

Among the different available techniques, the visible and near infrared spectroscopy (vis/NIR) has become popular in the field of vegetable matrices as a valid instrument for quality monitoring.

Optical instruments currently on the market are mainly laboratory instruments with such sizes and costs that they would not be applicable in the field. To solve this problem, in recent years research has focused on feasibility studies and simulations of small-sized simplified systems based on the analysis of the information contained in the spectra (optical footprint of the sample after interaction with the light) of samples in the spectral region of visible and near infrared. These studies are preparatory for designing systems targeted at individual product types, which are small-sized and cost-effective. In parallel, the development and distribution of low-cost, increasingly performing hardware systems has led to new research opportunities involving the use of these devices as new systems to support optical measurement for controlling and managing the same, and for real time sharing of acquired data by means of the cloud data.

The development of new simple and mutually interconnected optical systems would further allow to create remote observatories at the service of producers on the progress of maturation and allow the constant updating and refinement of the estimation performances of prediction models aimed at their continuous improvement for the benefit of the entire chain of plant production.

In order to understand the advantages of the result of the invention it is important to know that the use of other marketed instruments based on vis/NIR spectroscopy implies statistical knowledge and the need to apply complex multivaried analysis techniques to process data and interpret results. On the contrary, the concept behind the innovative result is an equipment capable of quickly estimating quality parameters (directly on location) which is easily usable by operators, instead of traditional spectra, allowing for a simple reading of results always known and used by the operator because they are measured with traditional laboratory analyses.

At the bibliographical level, we can find applications of non-destructive (high-cost) technologies mainly used in research centres where optical bench equipment and complex statistical algorithms (chemometry) are used to reach an estimation of quality of agri-food products, while at the commercial level there are low-cost optical technologies based on indexes specifically developed for very limited case studies (e.g. to determine maturation of apples and peaches only).

Currently, applications of ideal concepts can be found in scientific literature, which however do not hint at any prospect of realization.

Some simplified devices based on simple indexes are present on the market, but they have significant costs and are solely able to provide classifications of homogeneous product lots.

There are more accurate instruments which dissuade the final user from using them because they are more expensive and difficult to use. Moreover, these instruments entail a complex step of statistical processing subsequent to data acquisition.

In the agri-food sector there is the need to conduct extensive controls throughout the whole stages of the chain, from the field to the processing stages up to the end consumer, but most of all there is the need to monitor throughout the most critical stages before and after harvest.

In this particular area, commercially available equipment fails to fully meet the needs of the market, due to being excessively expensive and complex on the one hand (laboratory or research equipment), due to the lack of simple, cost-effective instruments targeted at specific applications on the other.

Furthermore, today marks a particular moment in the agri-food sector, characterized by a clear-cut generational (and cultural) shift between "old" farmers, relying on first-hand experience, and "new" young farmers, technologically savvy and attracted to technology applied to the specific work area of interest. In this scenario, the will and need for an instrument aid is conceived specifically to compensate for the lack of experience, aiming to objectify what was evaluated subjectively as far as instruments are concerned.

Some of the drawbacks of the prior art are:
- Complexity of the hardware and already marketed optical instruments difficult to use;
- Difficulty in identifying the most suitable solution according to the specific application and the matrix to be analysed;
- Cost of already marketed optical instruments being too high and not always sustainable according to the expected benefits deriving from their potential use;
- Initial setup of already marketed optical instruments being time-consuming and requiring technical knowledge and potential need to develop dedicated accessories for the application;
- Optical technology and chemometrics experts needed for the use of the equipment;
- Scepticism towards innovative optical technology, *black box* or *magic box* effect as compared to consolidated laboratory equipment.

WO 2019/236843 A1 discloses systems and methods for distinguishing fertile plant specimens from sterile plant specimens.

US 2010/182604 A1 discloses an optoelectric device for measuring the water content in a plant element and an apparatus designed to evaluate and monitor in real time the state of hydration of the plant covers.

### Object of the invention

In this context, the technical task of the present invention is to propose a portable device for analysing vegetable matrices in the field in order to overcome at least some drawbacks of the above-mentioned prior art.

Specifically, an object of the present invention is to provide a portable device for analysing vegetable matrices in the field, which is reliable and easy to use, also for users less experienced in the field.

A further object of the present invention is to provide an affordable device.

Another object of the present invention is to make a portable device which allows to analyse vegetable matrices throughout all the stages of the agri-food chain, from the field to the processing stages, up to the product ready for consumption (e.g. application at the point of sale), but most of all the invention is aimed at monitoring throughout the most critical stages before and after harvest, e.g. at (i) monitoring maturation, (ii) identifying optimal time for harvest, (iii) carrying out a screening of goods upon delivery, (iv) carrying out a selection of maturation classes for a better management of storage and shelf life also at the large-scale distribution or logistics platforms, (v) analysing the water and phytosanitary status of crops directly in the field, (vi) analysing qualitative aspects of the feed such as moisture content.

The invention obtains, in general, the following technical effects:
- it allows to analyse in real time vegetable matrices in the field, with an easy-to-use, low-cost portable device;
- it allows to conduct extensive controls throughout the whole stages of the chain, from the field to the processing stages up to the end consumer, but most of all to monitor throughout the most critical stages before and after harvest;
- it allows to provide for a simple and reliable device;
- allows to provide for a device which can be used by users with little experience in the agri-food sector;
- it allows for a reduced power consumption and thus an increased battery life;
- it allows to conduct a measurement setup and a remote firmware update.
The technical effects/advantages mentioned and other technical effects/advantages will result in more detail from the following description of an indicative and non-limiting example of an embodiment with reference to the attached drawings.

### Brief description of the drawings

In order to better understand the invention and value its advantages, some exemplary and non-limiting embodiments thereof will be described as follows, with reference to the attached drawings, wherein:
- figure 1 shows the portable device for analysing vegetable matrices according to the invention;
- figure 2 shows a detail of the device of figure 1 ;
- figure 3 shows a sensor used in the device of figures 1 and 2;
- figure 4 shows a block diagram of the operation of the device.

### Detailed description of preferred embodiments of the invention

In a **first** aspect, the present invention relates to a portable device indicated in the attached figures with reference number 1.

The portable device 1 for analysing vegetable matrices VM according to the present invention comprises a support body 2, configured to embrace the vegetable matrix VM to be analysed, an optical measurement element 10 and an electrical power source configured to power the various elements of the device 1.

Preferably, the optical measurement element 10 defines a single body together with the support body 2, specifically a single casing.

The support body 2 has a substantially longitudinal extension and is handleable on one end by a user's hand and has an element 3a, 3b configured to completely embrace within it the vegetable matrix VM to be analysed.

Specifically, the device illustrated in the drawings for non-limiting purposes is able to analyse vegetable matrices of fruits (grapes, cherries, blueberries, raspberries, blackberries, etc.) or small-sized vegetables (olives).

Preferably, the element 3 configured to embrace the vegetable matrix VM consists of two elements 3a, 3b, which are mutually movable between a maximum reciprocal distance, such as to allow the passage of the vegetable matrix VM between them, and a minimum distance, such as to allow the vegetable matrix VM to be embraced completely within them, thus forming a sort of dark chamber which allows for an optimal acquisition of optical data, free from reflected light signal disturbances.

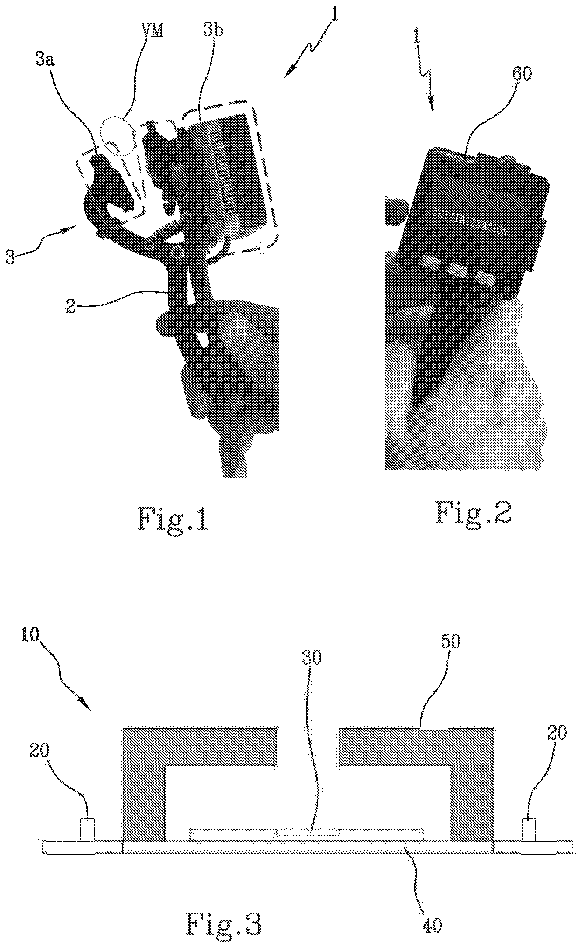

As illustrated in figures 1 and 2, the support body 2 has an extension along a longitudinal axis and comprises the two movable elements 3a, 3b in a first end and two movable levers handleable by a hand in the second longitudinally opposed end.

The two movable elements 3a, 3b are hinged proximate to the two handleable levers.

At least one movable element 3b is integral with one of the two levers of the handle, preferably made of a single piece. In this way, the whole portable device 1 turns out to be a handheld device, i.e. practically fitting in the palm of a hand, thus allowing for a high portability and usability in the field by a single operator.

The measurement element 10 is configured for a spectral measurement of optical data, in a given wavelength range of the vegetable matrix VM so as to acquire optical reflectance OR data of the surface of the vegetable matrix VM.

The optical measurement element 10 is configured for a measurement of wavelengths from 400 nm to 1,000 nm, preferably from 450 to 860 nm and comprises at least one light source 20 configured to generate an optical beam that is direct and incident on the surface of the vegetable matrix VM (present in the housing 3a, 3b) and an optoelectronic sensor 30 configured to acquire the optical data.

Specifically, the light source consists of one or more Light-Emitting Diodes (LEDs), while the optoelectronic sensor is, for example, a photodiode.

The light source 20 and the optoelectronic sensor 30 are formed monolithically on a support plate 40 -MEMS.

The optical measurement element 10 also comprises a protection element 50 configured to focus and receive the optical beam and protect the optoelectronic sensor 30 from direct lighting generated by the light source 20 and from ambient light external to the device 1.

The protection element 50 is perpendicular to the surface of the support plate 40 and is placed between the leds of the light source 20 and the optoelectronic sensor 30.

The protection element 50 is closed on the four sides perpendicular to the plate 40 and has a surface which is distanced from and parallel to the plate 40 with an opening adapted to let the reflected light pass through it and conveying it towards the optoelectronic sensor.

Thus, the interference of the light source with the optoelectronic sensor 30 is avoided.

Preferably, the protection element 50 is made of a flexible material and projects with respect to the surface of the support plate 40 on which the light source 20 and the optical sensor 30 are housed, so as to perfectly adhere to the surface of the vegetable matrix VM during analysis and detection.

Specifically, the optical device incorporates MEMS sensors equipped with multi-channel digital sensors, each of them being configured for spectral measurement of the visible and "Short Wave Near-Infrared" (SW-NIR) region.

In other words, the optical measurement element 10 is a MEMS sensor comprising one or more LEDs and provided with multi-channel digital sensors.

Preferably, the device 1 according to the present invention advantageously comprises one or more six-channel optical sensors.

For example, the device may comprise a first sensor operating in the wavelengths of 450, 500, 550, 570, 600 and 650 nanometers and a second sensor operating in the wavelengths of 610, 680, 730, 760, 810 and 860 nanometers.

In this case, the sensors cover overall 12 independent optical filters on the device from 450 to 860 nanometers.

The presence of the visible field (400-700 nanometers) makes the device 1 useful to obtain information also on the colour, an essential feature of the maturation process and for the acceptability of products at the time of harvest.

The portable device 1 also comprises a processing unit configured to receive optical reflectance OR data acquired by the measurement element 10, to process such data and generate a signal representative of at least one characteristic chemical-physical parameter Pi of the vegetable matrix VM or generate a classification of the analysed vegetable matrix VM on the basis of maturation classes, or in general on the basis of classes deriving from the evolution of qualitative parameters (for example sugar content, acidity, water, fats, etc.).

The inventive result is placed in the area of low-cost dedicated sensor equipment for the estimation of qualitative (chemical-physical) parameters of vegetable matrices VM such as for example soluble solids content (°Brix), acidity, pH, polyphenol, carotenoid and chlorophyll content for fruits such as grape, blueberry, tomato, or water content for vegetables such as salad and feed, or oil content for olives.

In general, it is to be noted that in this context and in the following claims, the processing unit will be intended as divided in distinct functional modules (memory modules or operational modules) for the only purpose of clearly and fully describing the functionalities thereof.

Such processing unit may consist of a single electronic device, suitably programmed to carry out the described functionalities, and the different modules may correspond to hardware entities and/or to software routines being part of the programmed device.

Alternatively or in addition, such functionalities may be carried out by a plurality of electronic devices on which the above-mentioned functional modules can be distributed.

The processing unit may further dispose of one or more processors for carrying out instructions contained in the memory modules.

The above-mentioned functional modules may further be distributed on different computers locally or remotely, depending on the architecture of the network in which they reside.

The signal representative of at least one characteristic chemical-physical parameter Pi of the vegetable matrix VM is estimated by means of chemometric models.

Optionally, the support body 2 of the portable device 1 may comprise display means 60 configured to display characteristic chemical-physical parameters Pi or the qualitative class of the analysed vegetable matrix VM and system information (for example acquisitions monitoring, battery level, signal coverage).

The display means 60 may define a single body together with the support body, specifically a single casing as illustrated in figures 1 and 2.

Specifically, the display means 60 are coupled to a back shell adapted to define a casing within which the optical sensor 10, the electric power source, the processing unit and possible means of connection with the outside of the portable device 1 are housed.

The electrical power source has the function of generating an internal battery continuous voltage signal V_{bat_i} to power the electronical components within the portable device 1, such as for example the processing unit or the optical measurement element 10. For example, the electrical power source is an electric battery of the Li-Ion ("Lithium-Ion Battery") type composed of cells connected in series, so as to generate (at the ends of the series of cells) an internal battery voltage V_{bat_i} having a value preferably between 3.3 and 5 Volts depending on the required autonomy range or transmission power.

The processing unit of the device allows the user to modify some parameters such as, for example, ID, sample name, repetitions of analyses, sample data acquisition times, etc.

The processing unit may comprise one or more of the following modules:
- a module configured to acquire the optical data acquired by the measurement element 10;
- a module configured to carry out pre-treatments of the optical data of the vegetable matrix VM acquired by the measurement element 10, such as for example smoothing, first derivative, autoscaling and the like;
- a module for carrying out the quantitative analysis or the classification analysis by means of suitable chemometric predictive models;
- a module for processing and generating a chemometric model to estimate the result;
- a module configured to transmit and/or receive data from outside the device 1.

One or more of the above modules can be distributed on the cloud and made accessible by means of Web application.

Advantageously, the portable device 1 comprises a connection module configured to transmit and receive signals.

Specifically, the connection module can be configured for a cable connection, for example, by means of a USB port or other equivalent connections.

In addition or alternatively, the connection module can be a long-distance or short-distance wireless signal transceiver or both and is powered by the power source.

The long-distance wireless signal transceiver is electrically connected to the processing unit and has the function of sending a long-distance wireless signal which transports a signal representative of the optical data detected by the measurement element 10 or the characteristic chemical-physical parameter Pi or the classification parameter of the vegetable matrix VM generated by the processing unit.

The long-distance wireless signal is for example a 2G, 3G, 4G or 5G or LoRa^{®} or Sigfox^{®} mobile radio transmitter signal.

The short-distance wireless signal transceiver is electrically connected to the processing unit and has the function of receiving a short-distance wireless signal which can transport both the optical data detected by the measurement element 10 and the characteristic chemical-physical parameter Pi or the classification parameter of the vegetable matrix VM generated by the processing unit.

The short-distance wireless signal is for example Bluetooth or WiFi.

The processing unit is electrically connected to the transceiver, to a memory unit and to a measurement element by means of a communication bus.

The processing unit is for example a microprocessor, a micro-controller, a programmable electronic circuit, or a dedicated integrated circuit.

Advantageously, the data connection module is configured to connect to an external electronic device, so that the optical data acquired by the portable device 1 can be displayed, stored and/or transmitted over long-distances. In this case, the external electronic device serves as a "hot-spot", Wi-Fi or Bluetooth for the portable device 1.

Furthermore, it is possible to display the parameters and the IP address of the external electronic device on the display of the portable device 1.

The external electronic device comprises at least one selected from the group of: a smartphone, a tablet, a smart-watch, a portable or desktop computer.

In a **second** aspect, the present invention relates to a system for analysing vegetable matrices VM in the field comprising at least one portable device 1 for analysing vegetable matrices VM as described above; connection means configured to send or receive data from the portable device 1 to an external electronic device, a remote server and/or a cloud.

If the portable device 1 is configured to send or receive data to at least one external electronic device connectable to the portable device 1, it is configured to:
- receive, as input, the optical data acquired by the measurement element 10 of the device 1;
- generate an output signal representative of the quantitative or classification analysis or a chemometric model for estimating the result of the analysed vegetable matrix VM.

Specifically, the external device comprises at least one or more of a smartphone, a tablet, a portable PC or a smart-watch.

Advantageously, the system comprises a network of long-distance telecommunications configured to connect the portable device 1 to one or more remote servers.

In a **third** aspect, the present invention relates to a method for analysing vegetable matrices VM in the field comprising the steps of providing a portable device 1 for analysing vegetable matrices VM in the field as described above; positioning the vegetable matrix VM in the housing between the movable elements 3a, 3b in order to proceed with optical data acquisition; starting the optical data acquisition through the measurement element 10; and, finally, generating a signal representative of at least one characteristic chemical-physical parameter Pi of the vegetable matrix VM or generating a classification of the analysed vegetable matrix VM on the basis of classes.

In case of a possible operation mode of the device 1 coupled with an external electronic device (for example a smartphone, a tablet, a portable PC, a smart-watch etc.) the device 1 can connect to the external electronic device activated as WiFi hot-spot. In this case, upon starting the portable device 1, the processing unit verifies whether the device is connected and ready to send and receive data with the outside by using the smartphone connection.

In another embodiment, the device 1 comprises means of connection to a network (for example, WiFi, Bluetooth, mobile phone network) to transmit and receive data from outside, for example, from a remote server. The acquired optical data, as well as the results of quantitative or classification analyses and related chemometric models for the estimation of the result, can be stored and/or processed on the cloud or on one or more proprietary servers.

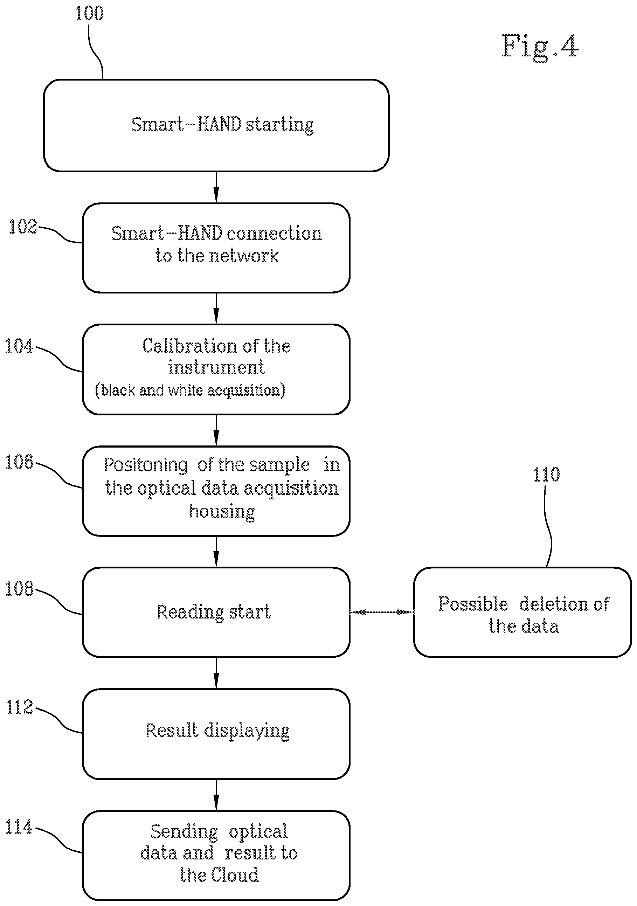

Figure 4 shows a block diagram of the operation of the device 1.

In the step 100, the device 1 is started by a user by means of a specific button. Once the device is started, it searches for a network connection (step 102).

Specifically, if the connection of the portable device 1 occurs by means of an external electronic device (for example a smartphone or the like), when the device 1 is first started, the device 1 functions in "hot spot" mode so as to allow the smartphone to connect thereto and to send username, password and other login parameters (since the portable device 1 might not be equipped with a keypad).

Optionally, it will be possible to display and verify the connection parameters on the device display. Once the device has acquired the connection parameters from the smartphone, the smartphone switches to "hot spot" operation mode, to which the device 1 is able to connect in order to transmit and receive data.

Then, there is a step 104 of calibration of the instrument, by means of reference optical standards acquisition (for example black and white) in order to standardize data acquisition.

Subsequently, in step 106, we proceed to place the vegetable matrix VM sample to be analysed between the two "clamp" elements 3a and 3b, so that they completely embrace the vegetable matrix, thus creating a sort of dark "chamber", completely isolated from possible external light sources and proceeding to the acquisition of the optical data through the sensors present in the measurement element 10. The acquired optical data is shown on the display 60 (step 112) and may be optionally deleted by the user (step 110).

Finally, the optical data and the chemical-physical parameter or the classification of the vegetable matrix may be sent to an external device, a remote server, or the Cloud.

Advantageously, the present invention achieves the proposed objects thus overcoming the drawbacks reported by the prior art by equipping the user with a low-cost portable device for analysing vegetable matrices in the field, related system and method, by means of which it is possible to obtain an estimation of chemical-physical parameters or a classification for qualitative monitoring of vegetable matrices throughout the entire arc of the agri-food chain (from the field to the end consumer).

## Claims

1. A portable device (1) for analysing vegetable matrices (VM) in the field, comprising:
- a support body (2) having a longitudinal extension and comprising:
∘ two movable elements (3a, 3b) in a first end of said support body, the two movable elements being configured to completely embrace a vegetable matrix (VM) to be analysed therein so as to create a dark chamber
∘ two movable levers handleable by a user's hand in a second end longitudinally opposed to the first end, said two movable elements (3a, 3b) being hinged proximate to the two handleable levers;
- a measurement element (10) configured for a spectral measurement in a given wavelength range of the vegetable matrix (VM), so as to acquire optical reflectance (OR) data of the surface of the vegetable matrix (VM);
- an electrical power source configured to power the various elements of the device;
- a processing unit configured to:
- receive said optical reflectance (OR) data acquired by the measurement element (10);
- generate a signal representative of at least one characteristic chemical-physical parameter (Pi) of the vegetable matrix (VM) or generate a classification of the analysed vegetable matrix (VM) on the basis of classes.

2. The device according to claim 1, wherein the two movable elements (3a, 3b) are movable between a maximum reciprocal distance, such to allow the passage of the vegetable matrix (VM) between them, and a minimum distance, such to allow the vegetable matrix (VM) to be embraced completely, thus forming the dark chamber.

3. The device according to claim 1 or 2, wherein said measurement element (10) is configured for a measurement of wavelengths from 400 nm to 1,000 nm, preferably from 450 to 860 nm.

4. The device according to one or more of the preceding claims, wherein said measurement element (10) comprises:
- at least one light source (20) configured to generate an optical beam that is incident on the surface of the vegetable matrix (VM);
- an optoelectronic sensor (30).

5. The device according to claim 4, wherein the light source (20) and the optoelectronic sensor (30) are formed monolithically on a support plate (40).

6. The device according to one or more of the preceding claims, comprising a flexible protection element (50) configured to focus and receive the optical beam.

7. The device according to claim 6, wherein said protection element (50) projects with respect to a surface of said support plate (40) and is flexible, so as to adhere to the surface of the vegetable matrix (VM).

8. The device according to one or more of the preceding claims, wherein said signal representative of at least one characteristic chemical-physical parameter (Pi) of the vegetable matrix (VM) is estimated by means of chemometric models.

9. The device according to one or more of the preceding claims, wherein said measurement element (10) is a MEMS sensor comprising one or more LEDs and provided with multi-channel digital sensors.

10. The device according to one or more of the preceding claims, wherein said support body (2) comprises display means (60).

11. The device according to one or more of the preceding claims, wherein said device (1) comprises signal connection means configured to transmit and receive data.

12. A system for analysing vegetable matrices (VM) in the field comprising:
- at least one portable device (1) for analysing vegetable matrices (VM) in accordance with any one of the preceding claims;
- connection means configured to send or receive data from the portable device (1) to an external electronic device, a remote server, or a cloud.

13. The system according to claim 12, wherein the portable device (1) is configured to send or receive data to at least one external electronic device connectable to the portable device (1) and configured to:
- receive, as input, the optical data acquired by the measurement element (10);
- generate an output signal representative of the quantitative or classification analysis or a chemometric model for estimating the result of the analysed vegetable matrix (VM);
wherein the external electronic device comprises at least one smartphone, a tablet, a portable PC or a smart-watch.

14. A method for analysing vegetable matrices (VM) in the field comprising the steps of:
- providing a portable device (1) for analysing vegetable matrices (VM) in accordance with any one of the preceding claims;
- positioning the vegetable matrix (VM) in the housing comprised between the two movable elements;
- starting the optical data acquisition;
- generating a signal representative of at least one characteristic chemical-physical parameter (Pi) of the vegetable matrix (VM) or generating a classification of the analysed vegetable matrix (VM) on the basis of classes.

## Patentansprüche

1. Tragbare Vorrichtung (1) zur Analyse pflanzlicher Matrizen (VM) auf dem Feld, umfassend:
- einen Stützkörper (2) mit einer Längserstreckung und umfassend:
∘ zwei bewegliche Elemente (3a, 3b) in einem ersten Ende des Stützkörpers, wobei die zwei beweglichen Elemente so ausgelegt sind, dass sie eine darin zu analysierende pflanzliche Matrix (VM) vollständig umschließen, um eine dunkle Kammer zu schaffen
∘ zwei bewegliche Hebel, die von der Hand eines Benutzers in einem zweiten Ende in Längsrichtung gegenüber dem ersten Ende gehandhabt werden können, wobei die zwei beweglichen Elemente (3a, 3b) in der Nähe der zwei handhabbaren Hebel angelenkt sind;
- ein Messelement (10), das für eine spektrale Messung in einem bestimmten Wellenlängenbereich der pflanzlichen Matrix (VM) ausgelegt ist, um optische Reflexions- (OR) Daten der Oberfläche der pflanzlichen Matrix (VM) zu erfassen;
- eine elektrische Stromquelle, die ausgelegt ist, um die verschiedenen Elemente der Vorrichtung mit Strom zu versorgen;
- eine Verarbeitungseinheit, die zu Folgendem ausgelegt ist:
- Empfangen der durch das Messelement (10) erfassten optischen Reflexions- (OR) Daten;
- Erzeugen eines für mindestens einen charakteristischen chemisch-physikalischen Parameter (Pi) der pflanzlichen Matrix (VM) repräsentativen Signals oder Erzeugen einer Klassifikation der analysierten pflanzlichen Matrix (VM) auf Basis von Klassen.

2. Vorrichtung nach Anspruch 1, wobei die zwei beweglichen Elemente (3a, 3b) zwischen einem maximalen gegenseitigen Abstand, um den Durchgang der pflanzlichen Matrix (VM) zwischen ihnen zu ermöglichen, und einem minimalen Abstand, um zu ermöglichen, dass die pflanzliche Matrix (VM) vollständig umschlossen wird, wodurch die dunkle Kammer gebildet wird, beweglich sind.

3. Vorrichtung nach Anspruch 1 oder 2, wobei das Messelement (10) für eine Messung von Wellenlängen von 400 nm bis 1.000 nm, vorzugsweise von 450 bis 860 nm, ausgelegt ist.

4. Vorrichtung nach einem oder mehreren der vorhergehenden Ansprüche, wobei das Messelement (10) Folgendes umfasst:
- mindestens eine Lichtquelle (20), die dazu ausgelegt ist, einen optischen Strahl zu erzeugen, der auf die Oberfläche der pflanzlichen Matrix (VM) auftrifft;
- einen optoelektronischen Sensor (30).

5. Vorrichtung nach Anspruch 4, wobei die Lichtquelle (20) und der optoelektronische Sensor (30) monolithisch auf einer Stützplatte (40) ausgebildet sind.

6. Vorrichtung nach einem oder mehreren der vorhergehenden Ansprüche, umfassend ein flexibles Schutzelement (50), das ausgelegt ist, um den optischen Strahl zu fokussieren und zu empfangen.

7. Vorrichtung nach Anspruch 6, wobei das Schutzelement (50) in Bezug auf eine Oberfläche der Stützplatte (40) vorsteht und flexibel ist, um an der Oberfläche der pflanzlichen Matrix (VM) zu haften.

8. Vorrichtung nach einem oder mehreren der vorhergehenden Ansprüche, wobei das für mindestens einen charakteristischen chemisch-physikalischen Parameter (Pi) der pflanzlichen Matrix (VM) repräsentative Signal mittels chemometrischer Modelle geschätzt wird.

9. Vorrichtung nach einem oder mehreren der vorhergehenden Ansprüche, wobei das Messelement (10) ein MEMS-Sensor ist, der eine oder mehrere LEDs umfasst und mit digitalen Mehrkanalsensoren versehen ist.

10. Vorrichtung nach einem oder mehreren der vorhergehenden Ansprüche, wobei der Stützkörper (2) Anzeigemittel (60) umfasst.

11. Vorrichtung nach einem oder mehreren der vorhergehenden Ansprüche, wobei die Vorrichtung (1) Signalverbindungsmittel umfasst, die zum Übermitteln und Empfangen von Daten ausgelegt sind.

12. System zur Analyse pflanzlicher Matrizen (VM) auf dem Feld, umfassend:
- mindestens eine tragbare Vorrichtung (1) zur Analyse pflanzlicher Matrizen (VM) nach einem der vorhergehenden Ansprüche;
- Verbindungsmittel, die dazu ausgelegt sind, Daten von der tragbaren Vorrichtung (1) an eine externe elektronische Vorrichtung, einen Remote-Server oder eine Cloud zu senden oder zu empfangen.

13. System nach Anspruch 12, wobei die tragbare Vorrichtung (1) dazu ausgelegt ist, Daten an mindestens eine externe elektronische Vorrichtung zu senden oder zu empfangen, die mit der tragbaren Vorrichtung (1) verbunden werden kann und zu Folgendem ausgelegt ist:
- Empfangen der durch das Messelement (10) erfassten optischen Daten als Eingabe;
- Erzeugen eines Ausgangssignals, das für die quantitative oder Klassifikationsanalyse repräsentativ ist, oder eines chemometrischen Modells zum Schätzen des Ergebnisses der analysierten pflanzlichen Matrix (VM),
wobei die externe elektronische Vorrichtung mindestens ein Smartphone, ein Tablet, einen tragbaren PC oder eine Smartwatch umfasst.

14. Verfahren zur Analyse pflanzlicher Matrizen (VM) auf dem Feld, umfassend die folgenden Schritte:
- Bereitstellen einer tragbaren Vorrichtung (1) zur Analyse pflanzlicher Matrizen (VM) nach einem der vorhergehenden Ansprüche;
- Positionieren der pflanzlichen Matrix (VM) in dem Gehäuse, das zwischen den zwei beweglichen Elementen enthalten ist;
- Starten der Erfassung der optischen Daten;
- Erzeugen eines für mindestens einen charakteristischen chemisch-physikalischen Parameter (Pi) der pflanzlichen Matrix (VM) repräsentativen Signals oder Erzeugen einer Klassifikation der analysierten pflanzlichen Matrix (VM) auf Basis von Klassen.

## Revendications

1. Dispositif portable (1) d'analyse de matrices végétales (VM) dans le terrain, comprenant :
- un corps de support (2) comportant une extension longitudinale et comprenant :
∘ deux éléments mobiles (3a, 3b) dans une première extrémité dudit corps de support, les deux éléments mobiles étant configurés pour englober complètement une matrice végétale (VM) à analyser de manière à créer une chambre noire
∘ deux leviers mobiles manipulables par la main d'un utilisateur dans une deuxième extrémité opposée longitudinalement à la première extrémité, lesdits deux éléments mobiles (3a, 3b) étant montés articulés à proximité des deux leviers manipulables ;
- un élément de mesure (10) configuré pour une mesure spectrale dans une gamme de longueurs d'onde donnée de la matrice végétale (VM), afin d'acquérir des données de réflectance optique (OR) de la surface de la matrice végétale (VM) ;
- une source d'énergie électrique configurée pour alimenter les différents éléments du dispositif ;
- une unité de traitement configurée pour :
- recevoir lesdites données de réflectance optique (RO) acquises par l'élément de mesure (10) ;
- générer un signal représentatif d'au moins un paramètre physico-chimique (Pi) caractéristique de la matrice végétale (VM) ou générer une classification de la matrice végétale (VM) analysée sur la base de classes.

2. Dispositif selon la revendication 1, dans lequel les deux éléments mobiles (3a, 3b) sont mobiles entre une distance réciproque maximale, de manière à permettre le passage de la matrice végétale (VM) entre eux, et une distance minimale, de manière à permettre d'englober complètement la matrice végétale (VM), formant ainsi la chambre noire.

3. Dispositif selon la revendication 1 ou 2, dans lequel ledit élément de mesure (10) est configuré pour une mesure des longueurs d'onde de 400 à 1 000 nm, de préférence de 450 à 860 nm.

4. Dispositif selon une ou plusieurs des revendications précédentes, dans lequel ledit élément de mesure (10) comprend :
- au moins une source lumineuse (20) configurée pour générer un faisceau optique étant incident sur la surface de la matrice végétale (VM) ;
- un capteur optoélectronique (30).

5. Dispositif selon la revendication 4, dans lequel la source lumineuse (20) et le capteur optoélectronique (30) sont formés de manière monolithique sur une plaque de support (40).

6. Dispositif selon une ou plusieurs des revendications précédentes, comprenant un élément de protection flexible (50) configuré pour focaliser et recevoir le faisceau optique.

7. Dispositif selon la revendication 6, dans lequel ledit élément de protection (50) dépasse par rapport à une surface de ladite plaque de support (40) et est flexible, de manière à adhérer à la surface de la matrice végétale (VM) .

8. Dispositif selon l'une ou plusieurs des revendications précédentes, dans lequel ledit signal représentatif d'au moins un paramètre chimico-physique (Pi) caractéristique de la matrice végétale (VM) est estimé au moyen de modèles chimiométriques.

9. Dispositif selon l'une ou plusieurs des revendications précédentes, dans lequel ledit élément de mesure (10) est un capteur MEMS comprenant une ou plusieurs LED et pourvu de capteurs numériques multicanaux.

10. Dispositif selon l'une ou plusieurs des revendications précédentes, dans lequel ledit corps de support (2) comprend des moyens d'affichage (60).

11. Dispositif selon l'une ou plusieurs des revendications précédentes, dans lequel ledit dispositif (1) comprend des moyens de connexion de signaux configurés pour transmettre et recevoir des données.

12. Système d'analyse de matrices végétales (VM) dans le terrain, comprenant :
- au moins un dispositif portable (1) d'analyse de matrices végétales (VM) selon l'une quelconque des revendications précédentes ;
- des moyens de connexion configurés pour envoyer ou recevoir des données du dispositif portable (1) vers un dispositif électronique externe, un serveur distant ou un cloud.

13. Système selon la revendication 12, dans lequel le dispositif portable (1) est configuré pour envoyer ou recevoir des données à au moins un dispositif électronique externe pouvant être connecté au dispositif portable (1) et configuré pour :
- recevoir, en entrée, les données optiques acquises par l'élément de mesure (10) ;
- générer un signal de sortie représentatif de l'analyse quantitative ou de classification ou d'un modèle chimiométrique pour estimer le résultat de la matrice végétale analysée (VM) ;
dans lequel le dispositif électronique externe comprend au moins un Smartphone, une tablette, un PC portable ou une montre connectée.

14. Méthode d'analyse de matrices végétales (VM) dans le terrain, comprenant les étapes de :
- fournir un dispositif portable (1) d'analyse de matrices végétales (VM) selon l'une quelconque des revendications précédentes ;
- positionner la matrice végétale (VM) dans le logement compris entre les deux éléments mobiles ;
- démarrer l'acquisition des données optiques ;
- générer un signal représentatif d'au moins un paramètre physico-chimique (Pi) caractéristique de la matrice végétale (VM) ou générer une classification de la matrice végétale (VM) analysée sur la base de classes.
